# EUROPEAN PATENT APPLICATION

(11) **EP 4 129 306 A1**
(43) Date of publication of application: **08.02.2023**
(21) Application number: 21780780.9
(22) Date of filing: 02.03.2021
(51) Int. Cl.: A61K 33/00, A23K 20/20, A23K 50/40, A23K 50/80, A23L 3/358, A23L 5/00, A23L 29/00, A23L 33/10, A61K 8/25, A61P 1/08, A61P 1/10, A61P 1/12, A61P 17/00, A61P 17/18, A61P 29/00, A61P 37/08, A61P 39/06, A61Q 19/00

(54) **OXIDATIVE STRESS INHIBITOR AND ANTIOXIDANT AGENT**

(30) Priority: 02.04.2020 JP 2020067000; 02.04.2020 JP 2020066999; 29.01.2021 WO PCT/JP2021/003383
(71) Applicant: BOSQUET SILICON CORP., Osaka-shi, Osaka 530-0001 (JP); KIT Co., Ltd., Kita-ku Osaka-shi Osaka 530-0001 (JP); Mitsui Mining & Smelting Co., Ltd., Shinagawa-ku Tokyo 141-8584 (JP)
(72) Inventor: KOBAYASHI Yuki, Kyoto-shi, Kyoto 605-0981 (JP)
(74) Representative: Moré, Solveig Helga
(86) International application number: PCT/JP2021/007917
(87) International publication number: WO 2021/199850

(57) **Abstract**

An oxidative stress inhibitor 100 of the present invention contains silicon particles capable of generating hydrogen. In a preferred aspect of the oxidative stress inhibitor 100, the following effects (a) and/or (b) can be exhibited. (a) The total amount of generation (total amount of production) is increased. (b) The amount of generation of hydrogen per unit time (i.e. rate of generation of hydrogen), particularly the rate of generation of hydrogen in the initial stage is increased.

## Description

### TECHNICAL FIELD

The present invention relates to an oxidative stress inhibitor and an antioxidant agent.

### BACKGROUND ART

Hydrogen is widely applied, and expected to be used in many applications. Examples thereof include targets caused by oxidative stress, and published involvement in growth. For example, in a body of a human, active oxygen produced in derived from oxygen generated in mitochondria in cells by metabolism in the body and subdermally under ultraviolet irradiation and derived from oxygen taken in from the lung is present. Active oxygen is known to oxidize and damage cells that form a living body while it is necessary for life support. Particularly a hydroxyl radical which has the strongest oxidizing power in active oxygen is considered to cause various diseases such as cancer, stroke, myocardial infarction, diabetes, other lifestyle diseases, and skin disorders such as skin aging and dermatitis. Therefore, it is desirable that excess active oxygen, particularly a hydroxyl radical, which has not been used in a reaction useful for a living body, be prevented from being present in the body wherever possible.

Regarding exclusively oxidative stress in a living body, hydroxyl radicals produced in the body are eliminated by reacting with some substances. Antioxidants in a living body, such as polyphenol, vitamin C, α-tocopherol or glutathione are generally considered as examples of substances that eliminate hydroxyl radicals. However, these substances eliminate not only hydroxyl radicals but also active oxygen having a function in the body, such as hydrogen peroxide, and thus may have adverse effects (side effects) such as a decrease in immunity. In addition, it is known that hydrogen can also eliminate hydroxyl radicals. However, hydrogen reacts only with hydroxyl radicals in active oxygen, and therefore does not have the above-described adverse effects (side effects). Thus, an apparatus for producing hydrogen water containing hydrogen that eliminates hydroxyl radicals in the body has been proposed (for example, Patent Document 1).

However, hydrogen (H₂) has an extremely low saturated solubility of 1.6 ppm in water at 25°C, and hydrogen in hydrogen water is easily diffused into air. Thus, for taking hydrogen in the body in an amount necessary for eliminating hydroxyl radicals, it is necessary that the concentration of dissolved hydrogen in hydrogen water be kept high. Therefore, with a method in which hydrogen water is ingested, it is impossible to take hydrogen in the body in an amount sufficient to react the hydrogen with hydroxyl radicals in the body. Thus, for easily taking hydrogen in the body, a hydrogen-containing composition containing hydrogen and a surfactant has been proposed (Patent Document 2), but it is not possible to keep the hydrogen concentration high in the body over a long period of time. Hydroxyl radicals have high reactivity, rapidly oxidize cells, and are constantly generated in a living body, and thus it is necessary to constantly supply high-concentration hydrogen into the body for eliminating the hydroxy radicals.

In view of the above-described background, a solid preparation has been disclosed which contains silicon fine particles as a main component, has high capability of generating hydrogen, and can be intended for oral use (Patent Document 3). In addition, a composite material has been disclosed which contains silicon fine particles, silicon suboxide (SiOx, where x is 1/2, 1, and 3/2) covering at least a part of surfaces of the silicon fine particles and/or a mixed composition of the silicon suboxide and silicon dioxide (Patent Document 4).

### PRIOR ART DOCUMENTS

### PATENT DOCUMENTS

Patent Document 1: Japanese Patent No. 5514140
Patent Document 2: Japanese Patent Laid-open Publication No. 2015-113331
Patent Document 3: International Publication WO 2017/130709
Patent Document 4: International Publication WO 2019/211960
Patent Document 5: International Publication WO 2018/037752
Patent Document 6: International Publication WO 2018/037818
Patent Document 7: International Publication WO 2018/037819

### NON-PATENT DOCUMENTS

Non-Patent Document 1: Matsuda et al., Water decomposition due to silicon nanoparticles and hydrogen concentrations, Extended Abstracts of the 62nd JSAP Spring Meeting, 2015, 12-031
Non-Patent Document 2: Fujinoe et al., Generation of hydrogen by reaction of silicon nanoparticles with neutral region, Extended Abstracts of the 64th JSAP Spring Meeting, 2015, 15a-421-6

### SUMMARY OF THE INVENTION

### PROBLEMS TO BE SOLVED BY THE INVENTION

However, even when hydrogen water is ingested, the amount of hydrogen contained in 1 liter of hydrogen water kept at 25°C is only 18 ml in terms of a gas. In addition, since hydrogen is the smallest molecule, is light, and has a high diffusion rate, it is impossible to thoroughly store hydrogen in a container containing the hydrogen water. A case of use in a living body will be described as an example. In the stomach, most of hydrogen in hydrogen water is gasified. Thus, there is the problem of causing pneumophagia (so-called "burp") because a sufficient amount of hydrogen is not taken in the body. Therefore, according to known information, it is impossible to keep the hydrogen concentration high in the body over a long period of time by ingesting hydrogen water and to ingest hydrogen water intermittently many times on a consecutive basis over a long period of time. On the other hand, when a hydrogen-containing composition with hydrogen encapsulated by a surfactant is ingested, it is impossible to take a sufficient amount of hydrogen in the body over a long period of time by ingesting the hydrogen-containing composition. In addition, there may arise the above-mentioned problem that hydrogen is released in the stomach. Further, assume that the hydrogen is used on the skin. Since use on the skin is no different from use in the air, hydrogen diffuses into the air and scatters in a short time because it diffuses at a much higher rate than in the body. Therefore absorption from the skin is difficult. In addition, regarding generation of hydrogen using the silicon fine particles which have been disclosed heretofore, there is still room for improvement in at least the amount of generation and the rate of generation of the hydrogen.

In addition, oxidative stress in a human living body which is caused by hydroxyl radicals is considered as one of causes of human aging. Therefore, the prevention or amelioration of human aging can increase the human healthy life expectancy to significantly contribute to not only individuals but also the improvement of the national medical economy typified by social security expenses such as medical care expenses.

### SOLUTIONS TO THE PROBLEMS

The present invention solves at least one of the technical problems described above, and can significantly contribute to more intense exhibition of the capability of generating hydrogen (production ability) of silicon particles or silicon fine particles. For example, it is possible to significantly contribute to rapidly generating a large amount of hydrogen depending on a situation or more reliably extracting a large amount of hydrogen in a human body, or in various moisture-containing spaces or water-containing liquids.

The present inventor has extensively conducted studies and analyses in order to improve human physical constitution, promote health, and treat or prevent various diseases by applying a substance capable of generating hydrogen. Regarding the generation of hydrogen, it is required to design a substance particularly in consideration of the rate of generation of hydrogen in the initial stage among the total amount of generation (total amount of production) and the amount of generation of hydrogen per unit time (i.e. rate of generation of hydrogen). Thus, for enhancing the capability of generating hydrogen, the present inventor has covered not only "silicon fine particles" including, as main particles, particles having an average crystallite diameter at a micron level or smaller, specifically a crystallite diameter of 1 nm or more and less than 1 µm, but also "silicon particles" having an average crystallite diameter of 1 µm or more, and conducted studies and analyses for finding a substance capable of exhibiting a high capability of generating hydrogen.

Thus, the present inventor has made an attempt to produce silicon particles or silicon fine particles (hereinafter, also referred to collectively as "silicon particles") using a method different from the production methods which have been heretofore employed. As one of the specific examples, the different production method is a production method including a classification step for obtaining silicon particles having relatively uniform particle sizes after a step of pulverizing silicon particles. In addition, while regarding silicon (Si) as one of elements, the present inventor has conducted studies including those on effects of other elements for enhancing the capability of generating hydrogen, as an example of another production method. Studies and analyses have been conducted for finding a substance capable of exhibiting a high capability of generating hydrogen by employing these production methods.

As a result of conducting experiments and analyses, the present inventor has found that silicon particles capable of generating hydrogen can play a role as an oxidative stress inhibitor. More preferably, the silicon particles include silicon particles having a crystallite diameter of 1 nm or more and 500 µm or less. In particular, it has been found that by employing silicon particles having the following characteristics (1) and/or (2), the capability of generating hydrogen is more intensively exhibited than before.
(1) The ratio of the silicon fine particles having a crystallite diameter of less than 1 µm and aggregates of the silicon fine particles having a crystallite diameter of less than 1 µm to all the silicon particles, the silicon fine particles and the aggregates thereof is 5 mass% or less.
(2) The silicon particles include a physiologically acceptable or medically acceptable metal (hereinafter, referred to collectively as a "physiologically acceptable metal") supported on, or attached or adsorbed to at least a part of surfaces of the silicon particles or the silicon fine particles, or chemically bonded to the surfaces, more specifically iron (Fe) or an iron (Fe)-containing substance (hereinafter, referred to collectively as "iron (Fe)").

More specifically, the present inventor has found that since the characteristics (1) and/or (2) are employed, the silicon particles are considerably superior in the total amount of generation (total amount of production) of hydrogen and/or the amount of generation of hydrogen per unit time in the initial stage to conventional silicon fine particles.

The results of analyses on the silicon particles (1) by the present inventor will be described in more detail. In the silicon particles (1), the abundance ratio of silicon particles of less than 1 µm and aggregates of the silicon particles to all silicon particles (and aggregates of the silicon particles) is low, so that the capability of generating hydrogen can be kept high, and a more stable capability of generating hydrogen can be exhibited because the crystallite diameter falls within a certain range. In addition, in the case of oral ingestion, deterioration of human mouthfeel can be suppressed. In addition, it is possible to reliably prevent silicon particles from being directly absorbed and entering the blood vessel.

The results of analyses on the silicon particles (2) by the present inventor will be described in more detail. As a result of analyzing the capability of generating hydrogen of silicon particles in which a specific metal element is supported on or attached or adsorbed to at least a part of surfaces of silicon particles or surfaces of the silicon fine particles, or the surfaces are chemically bonded to the metal, it has been found that the silicon particles more intensely exhibit the capability of generating hydrogen. An interesting finding has been obtained that employment of the silicon particles (2) increases the amount of generation of hydrogen, and resultantly, after the hydrogen generation reaction, the thickness of silicon suboxide (SiOx, where x is 1/2, 1, and 3/2; the same applies hereinafter) covering at least a part of surfaces of the silicon particles or the like and/or the thickness of the mixed composition of the silicon suboxide and silicon dioxide are larger than the thickness for conventional silicon fine particles. In particular, it is worth noting that the capability of generating hydrogen is intensely exhibited even if the silicon particles (2) are the silicon particles (1) including relatively large particles. In the present application, the "surface" of a silicon particle means the surface of a silicon suboxide film covering at least a part of the surface of the silicon fine particle or the silicon particle when the silicon particle includes the silicon suboxide film, the surface of a film of mixed composition of the silicon suboxide and silicon dioxide when the silicon particle includes the surface of the film of the mixed composition, or the surface of a natural oxide film when the silicon particle includes the natural oxide film.

The present invention has been made on the basis of the above-mentioned points of viewpoint.

The "silicon fine particles" in the present application include, as main particles, particles having an average crystallite diameter of 1 nm or more and less than 1 µm. In a narrower sense, the "silicon fine particles" in the present application include, as main particles, silicon nanoparticles having an average crystallite diameter at a nano level, specifically a crystalline diameter of 1 nm or more and 500 nm or less. In addition, the "silicon particles" in the present application include, as main particles, particles having an average crystallite diameter of more than 500 nm (in a narrow sense, 1 µm or more) and 500 µm or less.

In addition, in the present application, the "silicon fine particles" include not only those in which silicon fine particles are dispersed, but also those in which a plurality of silicon fine particles are aggregated to form aggregates in a µm order (generally 0.1 µm or more). Each of the above-described numerical value ranges for "silicon fine particles" is merely an example, and the numerical value range is not limited. In addition, the crystallite diameter is appropriately selected depending on the application, use method, required function and the like of the "silicon fine particles" or the "silicon particles".

In addition, the "water-containing liquid" in the present application is water or an aqueous solution, and encompasses gastrointestinal tract internal fluid of a human. The "gastrointestinal tract internal fluid" refers to small intestine internal fluid and large intestine internal fluid. It goes without saying that the example of the "water-containing liquid" is not limited to the above-described example. In addition, the material of the "pH adjusting agent" (hereinafter, referred to as an "alkali agent") in the present application is not particularly limited as long as it is an agent capable of adjusting the pH value to fall within an alkali range of more than 7 (typically more than 7.4). In addition, use on the skins of a human is included.

When an oxidative stress inhibitor of the present application as described later is used as an active oxygen neutralizing agent in a living body, it is preferable to use an alkali agent approved as any of a medicament (Pharmacopoeia drug), a quasi-pharmaceutical product and a food additive. Examples of the alkali agent that can be employed include sodium hydrogencarbonate, sodium carbonate, sodium dihydrogen phosphate, disodium hydrogenphosphate, potassium hydrogencarbonate, potassium carbonate, and pH adjusting agents for medicaments, quasi-pharmaceutical products, or food products. Sodium hydrogencarbonate which is the most versatile of the above-mentioned alkali agents is widely used for medicaments, quasi-pharmaceutical products or food additives because sodium hydrogencarbonate has a plurality of advantages such that it has a pH value adjustment function required in the present invention, and is excellent in safety and versatility. For any of the pH adjusting agents, a form in which the pH adjusting agent is not decomposed by an acid is a preferred aspect. In particular, when the oxidative stress inhibitor of the present application is orally ingested, a form is preferable in which oxidative stress inhibitor is not decomposed or is hardly decomposed by gastric acid.

An oxidative stress inhibitor of the present invention contains silicon particles and silicon fine particles capable of generating hydrogen.

This oxidative stress inhibitor can suppress oxidative stress in a human living body which is caused by hydroxyl radicals. In a preferred aspect of the oxidative stress inhibitor, at least one of the following effects (a) to (c) can be exhibited.
(a) The total amount of generation (total amount of production) is increased.
(b) The amount of generation of hydrogen per unit time (i.e. rate of generation of hydrogen), particularly the rate of generation of hydrogen in the initial stage is increased.
(c) It is possible to reliably prevent silicon particles from being directly absorbed and entering the blood vessel when orally ingested.

For example, in the invention described above, an oxidative stress inhibitor having the silicon particles in which the ratio of the silicon fine particles having a crystallite diameter of less than 1 µm and aggregates of the silicon fine particles having a crystallite diameter of less than 1 µm to all the silicon particles, the silicon fine particles and the aggregates thereof is 5 mass% or less (more preferably 3 mass% or less, still more preferably 1 mass% or less, still more preferably 0.5 mass% or less, even more preferably 0.2 mass% or less) can exhibit the effect of at least one of (a) to (c) above with higher reliability.

In addition, for example, in the invention described above, the oxidative stress inhibitor having iron (Fe) supported on, or attached or adsorbed to at least a part of surfaces of the silicon particles or silicon fine particles, or chemically bonded to the surfaces can exhibit the effect of at least one of (a) to (c) above with higher reliability.

Here, detailed mechanisms is still unknown, but support, attachment or adsorption (hereinafter, referred to collectively as "support") of a metal element increases the amount of generation of hydrogen due to the catalytic action of the metal element, and as a result, the thickness of silicon suboxide covering at least a part of silicon fine particles or silicon particles and/or the thickness of a mixed composition of the silicon suboxide and silicon dioxide can be made larger than the thickness for conventional silicon fine particles after the hydrogen generation reaction. That is, by employing this oxidative stress inhibitor, a reaction can more reliably occur in which silicon and water (or water in a water-containing liquid) are brought into contact with each other to form hydrogen, thereby forming silicon oxide. As a result, it is possible to exhibit a capability of generating hydrogen much higher than that of silicon particles or silicon fine particles on which the metal element is hardly supported (or not supported) or silicon particles or silicon fine particles which are hardly chemically bonded (or not chemically bonded) to the metal element. In particular, it is worth noting that the capability of generating hydrogen is intensely exhibited even in silicon particles having a crystallite diameter of 1 µm or more, which are relatively large particles.

In addition, according to the studies and analyses by the present inventor, an oxidative stress inhibitor of the present invention can contribute to maintenance or enhancement of human health or prevention or amelioration of human aging, and play a role as, for example, a supplement for preventing or ameliorating the following diseases or conditions (including symptoms) (1) to (5), a food product (including food additives and health foods), or a preparation for preventing or treating the following diseases or conditions (including symptoms) (1) to (5).
(1) Skin diseases
(2) Itching of skin
(3) Constipation or diarrhea
(4) Hangover syndrome
(5) Prevention or amelioration of human aging

The "skin disease" includes at least one selected from the group of eczema, inflammatory skin disease, allergic dermatitis with impaired skin barrier function, contact dermatitis, and atopic dermatitis.

### EFFECTS OF THE INVENTION

An oxidative stress inhibitor of the present invention is excellent in the total amount of generation (total amount of production) of hydrogen and/or the amount of generation of hydrogen per unit time in the initial stage.

### BRIEF DESCRIPTION OF THE DRAWINGS

Fig. 1(a) is a schematic view of an oxidative stress inhibitor according to a first embodiment, and Fig. 1(b) is a schematic view of an oxidative stress inhibitor according to a modification (1) of the first embodiment.
Fig. 2 is a graph showing a temporal change of hydrogen generated by reactions oxidative stress inhibitors of Exampled 1 and 2 and a comparative example with a water-containing liquid.
Fig. 3 shows cross-sectional TEM images showing silicon oxide thicknesses for an oxidative stress inhibitor (a) of Example 1 and a comparative example (b).
Fig. 4(a) is a photograph showing a state of a palm of a subject before ingestion of an oxidative stress inhibitor, and Fig. 4(b) is a photograph showing a state of the palm of the subject after continuous ingestion of the oxidative stress inhibitor for 5 days at 1 g per day, in examination of the effect of the oxidative stress inhibitor.

### EMBODIMENTS OF THE INVENTION

Embodiments of the present invention will be described on the basis of the accompanying drawings. In this description, like parts are given like reference symbols throughout the drawings unless otherwise specified. Further, in the drawings, elements of the embodiments are not necessarily shown on an actual scale. In addition, some reference symbols may be omitted for facilitating visualization of the drawings.

### [1] Oxidative stress inhibitor and method for production thereof

### FIRST EMBODIMENT

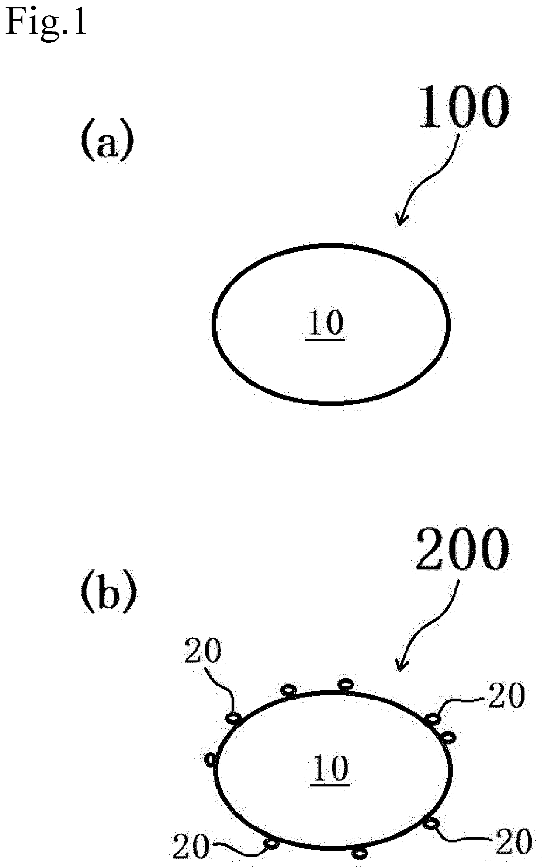

An oxidative stress inhibitor 100 according to the present embodiment, and a method for producing the oxidative stress inhibitor 100 will be described in detail. Fig. 1(a) is a schematic view of the oxidative stress inhibitor 100 according to the present embodiment. As shown in Fig. 1(a), the oxidative stress inhibitor 100 according to the present embodiment includes silicon particles or silicon fine particles (hereinafter, referred to collectively as "silicon particles") 10 capable of generating hydrogen.

A preferred aspect of the oxidative stress inhibitor 100 includes silicon particles 10 and silicon fine particles 10, in which the ratio of the silicon fine particles having a crystallite diameter of less than 1 µm and aggregates of the silicon fine particles having a crystallite diameter of less than 1 µm to all the silicon particles, the silicon fine particles and the aggregates thereof is 5 mass% or less (more preferably 3 mass% or less, still more preferably 1 mass% or less, still more preferably 0.5 mass% or less, even more preferably 0.2 mass% or less).

### [Method for producing oxidative stress inhibitor 100]

Next, the method for producing the oxidative stress inhibitor 100 according to the present embodiment. In addition, a surface of the oxidative stress inhibitor 100 measured or observed in the process of reaction of the oxidative stress inhibitor 100 with a water-containing liquid, and a silicon oxide film covering the surface will be described.

### <Pulverization step>

In the present embodiment, for example, commercially available high-purity silicon particle powder (particle size: 300 µm or less, purity: 99.999%, i-type silicon) is used as a part of raw materials for the oxidative stress inhibitor 100. In another aspect of the present embodiment, silicon particle powder having a purity higher than or lower than the aforementioned purity can be employed.

First, the high-purity silicon particle powder is subjected to pulverization treatment (first pulverization step) by a jet mill method using a cutter. A modification of the present embodiment in which after the first pulverization step, an additional pulverization step (second pulverization step) using zirconia beads of 0.5 mmϕ in ethanol is carried out in a bead mill method to obtain silicon fine particles (silicon nanoparticles) 10 having a representative crystallite diameter of less than 500 nm is also an aspect that can be employed. Further, an aspect in which instead of the first pulverization step, a roller mill method, a high-speed rotation pulverization method or a container driving mill method is used to obtain silicon particles 10 having a crystallite diameter of 1 µm or more and 60 µm or less or silicon fine particles (silicon nanoparticles) 10 having a representative crystallite diameter of less than 500 nm is an aspect that can be employed.

Employment of a mixed solution of ethanol at 99% or more (e.g. 99.5 wt%) and a small amount of water (e.g. 0.1 wt% or more and 10 wt% or less, more preferably more than 1 wt% and 2 wt% or less) in the case performing wet treatment in each of the pulverization steps is a preferred aspect from the viewpoint of enhancing the capability of generating hydrogen of the oxidative stress inhibitor 100 containing silicon particles 10.

### <Classification step>

Carrying out a classification step between the pulverization step (first pulverization step or second pulverization step) in the present embodiment and a metal element supporting step as described later is also an aspect of the present embodiment. Specifically, substantially all of silicon fine particles 10 having a crystallite diameter of less than 1 µm and aggregates of the silicon fine particles 10 having a crystallite diameter of less than 1 µm are removed using an airflow method so that the ratio of silicon fine particles 10 having a crystallite diameter of less than 1 µm and aggregates of the silicon fine particles 10 having a crystallite diameter of less than 1 µm to all the silicon particles 10, the silicon fine particles 10 and the aggregates thereof is 5 mass% or less (more preferably 3 mass% or less, still more preferably 1 mass% or less, still more preferably 0.5 mass% or less, even more preferably 0.2 mass% or less). The term "crystallite diameter" is employed for the silicon particle 10, whereas for the aggregate of silicon particles 10, the term "crystal grain diameter" which means the diameter of the entire aggregate is used.

When the oxidative stress inhibitor 100 capable of generating hydrogen in the present embodiment contains silicon particles 10 and silicon fine particles 10, and the ratio of silicon fine particles 10 having a crystallite diameter of less than 1 µm and aggregates of the silicon fine particles 10 having a crystallite diameter of less than 1 µm to all the silicon particles 10, the silicon fine particles 10 and the aggregates thereof is 5 mass% or less (more preferably 3 mass% or less, still more preferably 1 mass% or less, still more preferably 0.5 mass% or less, even more preferably 0.2 mass% or less), an effect can be exhibited such that safety can be enhanced by more reliably preventing passage of the oxidative stress inhibitor 100 through the cell membranes and between cells of the intestinal tract.

The upper limit of the crystallite diameter of silicon particles 10 and aggregates of silicon particles 10 is not limited as long as they have a capability of generating hydrogen. However, in addition to the classification step described above, a classification step carried out to remove substantially all of silicon particles 10 having a crystallite diameter of more than 60 µm or more preferably more than 45 µm and aggregates of the silicon particles 10 can be employed. More specifically, a classification step of performing classification so that the ratio of the silicon particles having a crystallite diameter of more than 60 µm (more preferably more than 45 µm) and aggregates of the silicon fine particles to all the silicon particles and the aggregates is 5 mass% or less (more preferably 3 mass% or less, still more preferably 1 mass% or less, even more preferably 0.5 mass% or less, even more preferably 0.2 mass% or less) can be employed. By removing substantially all of silicon particles 10 having a crystallite diameter of more than 60 µm or more preferably more than 45 µm and aggregates of the silicon particles 10, the capability of generating hydrogen can be kept high, and a more stable capability of generating hydrogen can be exhibited because the crystallite diameter falls within a certain range. In addition, in the case of oral ingestion, deterioration of human mouthfeel can be suppressed. In addition, it is possible to reliably prevent silicon particles from being directly absorbed and entering the blood vessel.

Resultantly, as an example, silicon particles 10 and aggregates of silicon particles 10 are obtained in which the ratio of the silicon fine particles having a crystallite diameter of less than 1 µm and aggregates of the silicon fine particles having a crystallite diameter of less than 1 µm to all the silicon particles, the silicon fine particles and the aggregates thereof is 5 mass% or less (more preferably 3 mass% or less, still more preferably 1 mass% or less, still more preferably 0.5 mass% or less, even more preferably 0.2 mass% or less). In another aspect, silicon particles 10 having a crystallite diameter of 1 µm or more and 60 µm or less (more preferably 1 µm or more and less than 45 µm) and aggregates of the silicon particles 10 is obtained.

Comparison of the oxidative stress inhibitor 100 according to the present embodiment with silicon particles which have not been subjected to a classification step according to the present embodiment shows that as described above, the oxidative stress inhibitor 100 according to the present embodiment is superior from the viewpoint of achieving a stable capability of generating hydrogen, suppressing deterioration of human mouthfeel upon oral ingestion, or reducing the possibility of ingress of silicon particles into the blood vessel. When silicon particles which have not been subjected to the classifying step are reacted with an aqueous sodium hydrogencarbonate solution (pH 8.3) at 37°C for 48 hours, the amount of generation of hydrogen is 295 mL/g. However, since the amount of generation of hydrogen by the oxidative stress inhibitor 100 under the same conditions as described above is 430 mL/g, it can be seen that the oxidative stress inhibitor 100 according to the present embodiment has a remarkably excellent capability of generating hydrogen.

By passing through the above-described steps, the oxidative stress inhibitor 100 including aggregates of the silicon particles 10 and the silicon particles 10 is obtained.

### <Modification (1) of first embodiment>

An oxidative stress inhibitor 200 according to the present modification, and a method for producing the oxidative stress inhibitor 200 will be described in detail. Fig. 1(b) is a schematic view of the oxidative stress inhibitor 200 according to the present modification. As shown in Fig. 1(b), the oxidative stress inhibitor 200 according to the present modification includes silicon particles or silicon fine particles (hereinafter, referred to collectively as "silicon particles") 10 capable of generating hydrogen, and a physiologically acceptable metal element 20 supported on, or attached or adsorbed to at least a part of surfaces of the silicon particles 10 or silicon fine particles 10, or chemically bonded to the surfaces.

In the present modification, the metal element 20 is iron (Fe). In addition, a state in which the metal element 20 is physically adsorbed on the silicon particles 10 is an example of a state in which the surfaces of the silicon particles 10 or the surfaces of the silicon fine particles 10 support the metal element 20. On the other hand, the state in which a part of the metal element 20 and the surfaces of the silicon particles 10 are chemically adsorbed and the state in which a compound (e.g. silicide) is formed in a region where the metal element 20 is in contact with the surfaces of the silicon particles 10 are an example in which the surfaces of the silicon particles 10 are chemically bonded to the metal element 20.

In addition, the amount of the metal element 20 supported on at least a part of the surfaces of the silicon particles 10 or silicon fine particles 10, or chemically bonded to the surfaces is not particularly limited. The typical amount of the metal element 20 is 0.1 ppmw or more and 1000 ppmw or less in terms of a mass ratio. From the viewpoint of obtaining a high capability of generating hydrogen including that in the initial stage (particularly within 2 to 3 hours after generation of hydrogen) as described later, the amount of the metal element 20 is preferably 0.5 ppmw or more (more preferably 1 ppmw or more, still more preferably 1.5 ppmw or more, even more preferably 2 ppmw or more) in terms of a mass ratio of the metal element 20, and the upper limit of the numerical range thereof is preferably 500 ppmw or less (more preferably 300 ppmw or more, still more preferably 100 ppmw or more, even more preferably 75 ppmw or less, even more preferably 50 ppm or less).

### [Method for producing oxidative stress inhibitor 200]

### <Step of supporting metal element>

With respect to the method for producing the oxidative stress inhibitor 100 according to the first embodiment, the method for producing the oxidative stress inhibitor 200 according to the present modification includes a step of supporting the metal element 20 on the surfaces of silicon particles 10 or the surfaces of silicon fine particles 10, or attaching or adsorbing the metal element 20 to the surfaces of silicon particles 10 or the surfaces of silicon fine particles 10, or a chemical bonding step of chemically bonding the metal element 20 and the surfaces of silicon particles 10 (hereinafter, referred to collectively as a "supporting step").

In an example of the supporting step in the present embodiment, spraying treatment using a spray apparatus for spraying a solution (e.g. an aqueous chloride solution) of the metal element 20 (e.g. iron (Fe)) to the silicon particles 10 that is being stirred is performed in a treatment chamber. For example, a spraying step of spraying a 10 mM aqueous FeCl₂ solution to silicon particles 10 is carried out. The solution of the metal element 20 is a part of raw materials for the oxidative stress inhibitor 200.

By passing through the supporting step described above, the oxidative stress inhibitor 200 according to the present modification can be produced.

In another example of the supporting step in the present embodiment, another step of bringing the silicon particles 10 into contact with the solution of the metal element 20 can be employed instead of the spraying step or together with the spraying step. As a specific example of the other step, a step of immersing silicon particles 10 in the solution of the metal element 20 manually or automatically by using a storage portion for storing the solution of the metal element 20 can be employed.

In another example of the supporting step in the present embodiment, a step of performing pulverization treatment using a SUS cutter in the pulverization step, whereby for example iron (Fe) is supported on or attached or adsorbed to the surfaces of silicon particles 10 or the surfaces of silicon fine particles 10 with the utilization of the composition of the cutter in the pulverization process. In that case, the pulverization step described above can also play a role as the supporting step in the present embodiment.

### <Analysis on generation of hydrogen by contact between oxidative stress inhibitor 200 and water-containing liquid>

According to the present inventor, the oxidative stress inhibitor 200 is then immersed in a water-containing liquid (pH value: 8.2) at a temperature equivalent to the human body temperature (37°C) which is an aqueous solution having sodium hydrogencarbonate as a pH adjusting agent as a solute in order to analyze the total amount of generation (total amount of production) of hydrogen (H₂) and the rate of generation (rate of production) of hydrogen when a contact step of bringing the water-containing liquid and the oxidative stress inhibitor 200 into contact with each other is carried out.

### <Examples>

Hereinafter, the first embodiment will be described more in detail by way of examples, but the first embodiment is not limited to these examples.

### [Example 1]

Example 1 is an oxidative stress inhibitor 200 in which a part of surfaces of silicon particles 10 or surfaces of silicon fine particles 10 support a metal element 20, or the surfaces are chemically bonded to the metal element 20. The metal element 20 in Example 1 is iron (Fe). In Example 1, the mass ratio of the metal element 20 to silicon particles 10 is about 10 ppmw.

### [Example 2]

Like Example 1, Example 2 is an oxidative stress inhibitor 200 in which a part of surfaces of silicon particles 10 or surfaces of silicon fine particles 10 support a metal element 20, or the surfaces are chemically bonded to the metal element 20. The metal element 20 in Example 2 is iron (Fe). In Example 2, the mass ratio of the metal element 20 to silicon particles 10 is about 5 ppmw.

As a comparative example, silicon particles produced through steps identical to those in Example 1 except that the supporting step in the modification (1) of the first embodiment was not carried out were employed. The mass ratio of the metal to the silicon particles in the comparative example is less than 0.1 ppmw, and in a more narrow sense, the mass ratio is equal to or less than the detection limit when an inductively coupled plasma mass spectrometer is used.

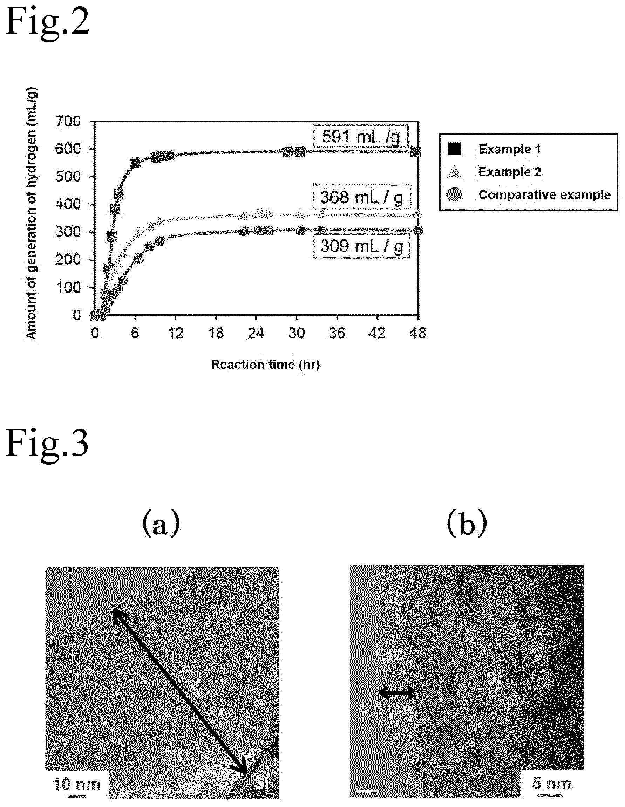

Fig. 2 is a graph showing a temporal change of hydrogen generated by reactions of the oxidative stress inhibitors 200 of Exampled 1 and 2 and the silicon particles of a comparative example with a water-containing liquid.

As shown in Fig. 2, it was revealed that for the oxidative stress inhibitor 200 of Example 1, a large amount of generation of hydrogen (591 mL (milliliter)/g (gram)), and a very large amount of hydrogen per unit mass (mL/g) immediately after the generation of hydrogen (particularly within 2 to 3 hours after generation of hydrogen) was obtained. Specifically, the amount of generation of hydrogen until 3 hours after generation of hydrogen was a very large value of about 385 mL/g. It is worth noting that a high capability of generating hydrogen can be exhibited over a long time of 40 hours or more (at least 48 hours in an example) after the start of generation of hydrogen as in Example 1.

In addition, it was revealed that for the oxidative stress inhibitor 200 of Example 2, a relatively large total amount of generation (368 mL/g), and a relatively large amount of hydrogen per unit mass (mL/g) immediately after the generation of hydrogen (particularly within 2 to 3 hours after generation of hydrogen) was obtained. Specifically, the amount of generation of hydrogen until 3 hours after generation of hydrogen was a relatively high value of about 170 mL/g. It is worth noting that a high capability of generating hydrogen can be exhibited over a long time of 40 hours or more (at least 48 hours in an example) after the start of generation of hydrogen as in Example 2.

As shown in Fig. 2, it was revealed that the comparative example in which the supporting step in the first embodiment had not been carried out showed an amount of generation of hydrogen (309 mL/g) smaller than that for the oxidative stress inhibitor 200, and the smallest amount of hydrogen per unit mass (mL/g) immediately after the generation of hydrogen (particularly within 2 to 3 hours after generation of hydrogen). Specifically, the amount of generation of hydrogen until 3 hours after generation of hydrogen was a low value of about 75 mL/g.

In order to confirm the reproducibility, the inventor of the present application examined the amount of hydrogen per unit mass (mL/g) for cases where iron (Fe) was employed as the metal element 20 as in Example 1, and the mass ratio of the metal element 20 to silicon particles 10 was set to about 0.5 ppmw, about 1.5 ppmw and about 2.0 ppmw, and the results showed that in each of the cases, the amount of hydrogen per unit mass (mL/g) (i.e. rate of generation of hydrogen) immediately after generation of hydrogen (particularly within 2 to 3 hours after generation of hydrogen) showed a value higher than that in the comparative example. Interestingly, in each of the above-described three types of mass ratios, values similar enough to be considered as falling within an experimental error range were obtained for the amount of hydrogen per unit mass (mL/g) immediately after generation of hydrogen and the amount of generation of hydrogen (mL/g) until completion of the hydrogen generation reaction.

### <Analysis with transmission electron microscope (TEM) image>

Further, using a transmission electron microscope (TEM), the present inventor analyzed the surface-covering silicon oxide film formed by the reaction between the oxidative stress inhibitor 200 of Example 1 and the water-containing liquid and the reaction between the silicon particles of the comparative example and the water-containing liquid. Fig. 3 shows cross-sectional TEM images showing silicon oxide thicknesses for an oxidative stress inhibitor (a) of Example 1 and a comparative example (b).

Very interestingly, from comparison between Figs. 3(a) and 3(b), it was confirmed that the thickness of the silicon oxide of the oxidative stress inhibitor 200 of Example 1 (about 113.9 nm) was 17 times or more the thickness of the silicon oxide of the comparative example (about 6.4 nm). At present, the detailed mechanism is not clear, but it is considered that a part of the metal element 20 on the surfaces of the silicon particles 10 exhibit a catalytic effect. It is considered that resultantly, as compared to a case where the metal element 20 is not present, the hydrogen generation reaction can be promoted, resulting in generation of a situation in which the thickness of silicon suboxide and/or the thickness of the mixed composition of silicon suboxide and silicon dioxide are easily increased (in other words, the hydrogen generation reaction is likely to occur).

Therefore, when the oxidative stress inhibitor 200 is employed in which the metal element 20 is supported on at least a part of surfaces of silicon particles 10 or surfaces of silicon fine particles 10, or the surfaces are chemically bonded to the metal element 20, the thickness of silicon suboxide covering at least a part of the surfaces of the silicon particles 10 and/or the thickness of the mixed composition of silicon suboxide and silicon dioxide are larger than the thickness for conventional silicon fine particles. The large thickness indicates that the reaction in which silicon and water (or water in a water-containing liquid) contact each other to form silicon oxide and hydrogen can more reliably occur as compared with conventional silicon fine particles.

As described above, employment of the oxidative stress inhibitor 200 in which at least a part of surfaces silicon particles 10 or surfaces of silicon fine particles 10 support the metal element 20 of which mass ratio to silicon particles 10 is in the numerical value range of at least 0.1 ppmw or more and 1000 ppmw or less or the surfaces are chemically bonded to the metal element 20 in the above-mentioned numerical value range can significantly contribute to more intensive exhibition of the capability of generating hydrogen of the oxidative stress inhibitor 200. For example, it is possible to significantly contribute to generating a large amount of hydrogen rapidly and over a long period of time depending on a situation or more reliably extracting a large amount of hydrogen in a human body, or in various moisture-containing spaces or water-containing liquids.

### <Modification (2) of first embodiment>

An aspect in which a reforming step of bringing the surfaces of silicon particles 10 or surfaces of silicon fine particles 10 into contact with hydrogen peroxide to reform the surfaces is carried out after the pulverization step is carried out in the first embodiment or after the pulverization step is carried out and before the supporting step is carried out in the modification (1) of the first embodiment is also a preferred aspect. By this reforming step, silicon particles including silicon nanoparticles can be made hydrophilic in a macroscopic point of view. For example, the reforming step can be carried out by immersing silicon particles in hydrogen peroxide water contained in a known container (about 10°C to about 80°C, and about 20°C to about 50°C from the viewpoint of further reducing the cost).

From the experiments by the present inventor, it has been confirmed that the amount of generation of hydrogen with the oxidative stress inhibitor 200 according to the modification (1) of the first embodiment (in which the reforming step using hydrogen peroxide water was not carried out) is larger than the amount of generation of hydrogen under conditions where the reforming step using hydrogen peroxide water is carried out and the supporting step in the modification (1) of the first embodiment is not carried out.

The same reforming can be performed by immersing the silicon particles in ozone water and/or sodium percarbonate instead of hydrogen peroxide water. Alternatively, the same reforming can be performed by bringing the silicon particles into contact with at least one selected from the group consisting of hydrogen peroxide water, ozone water and sodium percarbonate.

As described above, it is also preferred to carry out the reforming step using hydrogen peroxide water at substantially room temperature from the viewpoint of performing treatment at low cost and safely. In addition, employment of hydrogen peroxide water in the reforming step of the present modification is a preferred aspect in that hydrogen can be generated by using a safer and more secure material (e.g. having less influence on the human body) as in the case of ethanol.

### <Modification (3) of first embodiment>

In the present modification, 500 mg of the oxidative stress inhibitors 100 or 200 according to the first embodiment or the modification (1) or (2) of the first embodiment is mixed with about 500 mg of sodium hydrogencarbonate powder (manufactured by Wako Pure Chemical Industries, Ltd., purity: 99.5%). The mixture can be kneaded, and formed into a tablet (e.g. a tablet for medicaments or quasi-pharmaceutical products) as a columnar lump body having a diameter of about 8 mm and a height of about 4 mm by a tableting method. The tablet is an example of a lump preparation. An aspect in which the oxidative stress inhibitor 100 or 200 having stable silicon suboxide and a pH adjusting agent such as sodium hydrogencarbonate are separately formed into nanocapsules, microcapsules or normal capsules insoluble under acidic conditions and soluble under basic conditions, or applied is a preferred aspect. By employing the aforementioned aspect, the preparation can be dissolved to promote a reaction of the oxidative stress inhibitor 100 or 200 with water in the presence of moisture under basic conditions while avoiding reaction in the presence of moisture under acidic conditions.

### <Modification (4) of first embodiment>

The oxidative stress inhibitor 100 or 200 according to the first embodiment or each of the modifications (1) to (3) of the first embodiment can be applied as, for example, a preparation (medicament) or a quasi-pharmaceutical product. In addition, the application thereof is not limited to tablets. For example, even when a capsule preparation with the oxidative stress inhibitor 100 or 200 encapsulated in a capsule is employed in place of a tablet, the same effect as the above-mentioned effect can be exhibited. The oxidative stress inhibitor 100 or 200 can generate much hydrogen when the oxidative stress inhibitor is in the powdery form having a large surface area rather than being in a lump form, but the oxidative stress inhibitor is orally ingested or taken through the anus easily when formed into a tablet or a capsule preparation. In addition, when the composite material is formed into a tablet or a capsule preparation, it maintains a lump form to some extent, but is increasingly disintegrated to assume a powdery form after passing through the stomach. Thus, in the stomach where it is desirable to suppress the hydrogen generation reaction, the surface area of the oxidative stress inhibitor 100 or 200 exposed to gastric fluid and/or gastric contents can be reduced, and in the small intestine and/or large intestine where it is desired to promote the hydrogen generation reaction, the surface area of the silicon nanoparticles exposed to the water-containing liquid can be increased.

In addition, the oxidative stress inhibitor 100 or 200 may be a granular preparation. The granular preparation assumes a powdery form in an earlier stage after being orally ingested or taken through the anus as compared to tablets and capsules. In the case of oral ingestion, however, since the pH value of gastric fluid is low (about 1.5), the preparation generates little hydrogen even when assuming a powdery form immediately after reaching the stomach, and generates hydrogen in the presence of water after passage through the stomach.

The oxidative stress inhibitor 100 or 200 may be a powdered preparation. The powdered preparation is easy to handle when the oxidative stress inhibitor 100 or 200 is used as a constituent component of a food product such as a health food product, e.g. a food additive. When the oxidative stress inhibitor is used as a food additive, silicon particles 10 having a crystallite diameter of 1 nm or more and 10 µm or less or 1 nm or more and 500 nm or less (in a narrower sense, 1 nm or more and 100 nm or less) may be mixed and used as the oxidative stress inhibitor 100 or 200. Preferably, the silicon particles 10 are contained in an amount of 1 mass% or more. The upper limit of the content of silicon particles 10 is not specified, but is preferably 40 mass% or less with consideration given to the taste.

In addition, an example of a covering layer applicable to a tablet is a known enteric material hardly soluble in the stomach, which is a coating agent that covers the outermost layer of the tablet. In addition, an example of a covering layer applicable to a capsule preparation is a capsule itself which encapsulates the oxidative stress inhibitor 100 or 200, and is produced from a known enteric material hardly soluble in the stomach.

As described above, an example of a preparation suitable as an application of the oxidative stress inhibitor 100 or 200 is a tablet which is a lump preparation which is easily orally ingested or taken through the anus in a sufficient amount, or a capsule preparation in which the powdery oxidative stress inhibitor 100 or 200 (which may include those formed into aggregates) are encapsulated in a capsule. When a tablet is employed, a disintegrating agent may be further included. For the disintegrating agent, a known material can be employed. In addition, an example of a more preferred disintegrating agent is an organic acid, and the most preferred example is citric acid. Here, the organic acid can also function as a binding agent that brings silicon particles 10 into a lump form. The oxidative stress inhibitor 100 or 200 can also be applied as, for example, granular, flaky and/or powdered food topping materials (typically, "rice seasoning") for each food item.

### <Modification (5) of first embodiment>

In addition, the oxidative stress inhibitor 100 or 200 according to the first embodiment or each of the modifications (1) to (4) of the first embodiment enables hydrogen to be taken into the body transdermally or transmucosally (including the skin itself or the mucosa itself) by, for example, using a "medium" that is brought into contact with the oxidative stress inhibitor 100 or 200. The material or the commercial product for the medium in the present modification is not particularly limited. As long as the medium is physiologically acceptable, the effects of the present modification can be exhibited. Therefore, a material including the oxidative stress inhibitor 100 or 200 and the medium that contacts the oxidative stress inhibitor 100 or 200 can exhibit a function as a hydrogen supply material.

Specifically, from the viewpoint of increasing the opportunity for a portion of a human to be brought into contact with water (or a water-containing liquid) or a medium containing the water (or the water-containing liquid) (hereinafter, also referred to collectively as a "medium") in a living scene, an example of a preferred medium is in the form of at least one selected from the group of liquid, gel, cream, paste, emulsion and mousse. Another example of a preferred medium is bathing water (bathing water which is preferably alkaline). Therefore, in an example of the present modification, the production of the bathing water is a method for producing the medium.

Bath water will be described in more detail. Typically, tap water is stored as bath water in a common bath (a bathtub in a public bathhouse, a public bathtub, and an indoor or outdoor bathtub installed by an inn). Before and after the bath water is stored, the oxidative stress inhibitor is placed or put in the bathtub, and a contact step of bringing the oxidative stress inhibitor 100 or 200 into contact with the bath water as a medium is carried out to generate hydrogen (H₂). Therefore, the oxidative stress inhibitor 100 or 200 of the present modification can be employed as a so-called bath agent.

Therefore, the hydrogen (H₂) generated by the contact step can be brought into contact with the skin and/or a mucous membrane of the bathing human via bathing water as a physiologically acceptable medium. As a result, according to the present modification, it is possible to take hydrogen (H₂) into the human body (including the skin itself or the mucous membrane itself) using means different from oral ingestion or uptake through the anus.

### <Examples>

### [Example 3]

### [Effect of oxidative stress inhibitor on skin disease]

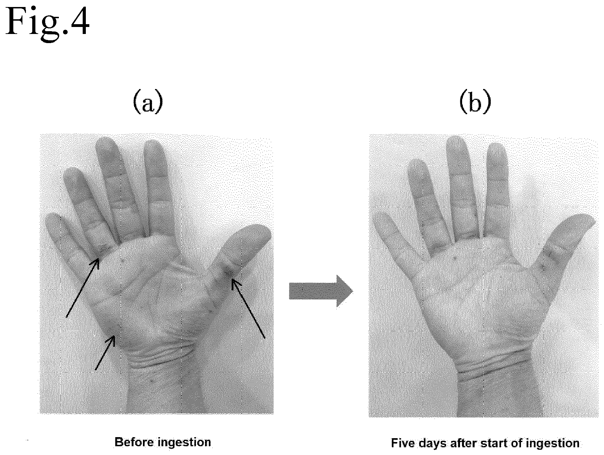

Fig. 4(a) is a photograph showing a state of a palm of a subject before ingestion of an oxidative stress inhibitor, and Fig. 4(b) is a photograph showing a state of the palm of the subject 5 days after ingestion of the oxidative stress inhibitor, in examination of the effect of the oxidative stress inhibitor.

In this example, a voluntarily participating subject (male in his thirties) having eczema and atopic dermatitis developed on a palm as indicated by the arrow in Fig. 4(a) orally ingested the oxidative stress inhibitor 100 every day at 1 g per day. The palm of the subject was observed 5 days after the start of ingestion of the oxidative stress inhibitor, and the result revealed that there was an evident ameliorating effect on both eczema and atopic dermatitis as shown in Fig. 4(b). According to the subject, itching of the hand was also ameliorated.

Amelioration of the conditions of eczema and atopic dermatitis on the hand by continuous ingestion of the oxidative stress inhibitor for only several days as shown in Fig. 4(b) is a very interesting finding and a noteworthy effect.

### [Example 4]

### [Effect of oxidative stress inhibitor on constipation or diarrhea]

In this example, subjects having a disease of constipation with a low frequency of bowel movement or diarrhea and voluntarily participating in this example (constipation: females in their twenties, forties and sixties) orally ingested the oxidative stress inhibitor 100 every day at 1 g per day. Whether or not bowel movement in the subject was improved and/or the number of healthy defecations 5 days after the start of ingestion of the oxidative stress inhibitor were examined, and the results revealed that there was an ameliorating effect on both the conditions.

Therefore, it can be said that an ameliorating effect on constipation and diarrhea was obtained by ingesting the oxidative stress inhibitor. In addition, the oxidative stress inhibitor can play a role as an agent for improving the human intestinal environment or a human intestinal regulator.

### [Example 5]

### [Effect of oxidative stress inhibitor on hangover syndrome]

In this example, subjects having a hangover syndrome (a series of physiological symptoms called "hangover") and voluntarily participating in this example (males in their thirties, forties, fifties and sixties) orally ingested the oxidative stress inhibitor 100 every day at 1 g per day. Whether or not the symptom of hangover in the subject was improved 2 days or more after the start of ingestion of the oxidative stress inhibitor was examined, and the results revealed that there was an ameliorating effect. The "hangover syndrome" includes phenomena of nausea, vomiting, headache, drowsiness, and decreased motion capacity. It is well known that acetaldehyde produced in the body during or after alcohol ingestion is a cause of the hangover syndrome.

Therefore, it can be said that an ameliorating effect on the hangover syndrome was obtained by ingesting the oxidative stress inhibitor.

### ANOTHER EMBODIMENT (1)

The oxidative stress inhibitor 100 or 200 according to the first embodiment or each of the modifications of the first embodiment can also be used as a human supplement or a food additive. A food product containing the oxidative stress inhibitor 100 or 200 according to the first embodiment or each of the modifications of the first embodiment is a preferred aspect that can be employed.

### ANOTHER EMBODIMENT (2)

In addition, the oxidative stress inhibitor 100 or 200 according to the first embodiment or each of the modifications of the first embodiment can be aggregated in a natural state to form aggregates having a diameter size of µm order (e.g. several tens of µm or more). The oxidative stress inhibitor 100 or 200 can be artificially put together by addition of the aggregate or a binding agent, compression or the like to form a formulation as a lump solid preparation having such a size that it can be picked up by human fingers.

### ANOTHER EMBODIMENT (3)

For example, the oxidative stress inhibitor 100 or 200 according to the first embodiment or each of the modifications of the first embodiment is brought into contact with a first water-containing liquid having a pH value of less than 7 in an initial contact step (a first contact step), and brought into contact with a second water-containing liquid having a pH value of 7 or more in a subsequent contact step (second contact step). In this way, hydrogen can be generated in the second contact step. The oxidative stress inhibitor 100 or 200 according to each of the embodiments can have a significant capability of generating hydrogen when coming into contact with a water-containing liquid having a pH value of 7 or more (more preferably more than 7, still more preferably 8.2 or more).

In addition, the temperature condition of the second water-containing liquid for generation of hydrogen is not limited. Possibly depending on the pH of the second water-containing liquid, generation of hydrogen can be promoted with high reliability as long as the temperature of the second water-containing liquid is 80°C or lower. However, the upper limit of the temperature of the second water-containing liquid is not limited. For example, when the oxidative stress inhibitor 100 or 200 according to each of the embodiments and modifications thereof is used as an industrial chemical, the temperature may exceed 50°C. However, when the temperature becomes higher, there is the problem that equipment (including a container) is required to have high heat resistance, and care is needed for handling. Therefore, the temperature is preferably 100°C or lower even when the solid preparation is used as an industrial chemical.

While a part of roles as a supplement for preventing or ameliorating the following diseases or conditions (including symptoms) (1) to (5), a food product (including a food additive and a health food product), or a preparation for preventing or treating the following diseases or conditions (including symptoms) (1) to (5) are described in the examples, the oxidative stress inhibitor 100 or 200 according to the first embodiment or each of the modifications thereof can play a role as a supplement for preventing or ameliorating all of the following diseases or conditions (including symptoms) (1) to (5), a food product (including a food additive and a health food product), or a preparation for preventing or treating the following diseases or conditions (including symptoms) (1) to (5).
(1) Skin diseases
(2) Itching of skin
(3) Constipation or diarrhea
(4) Hangover syndrome
(5) Prevention or amelioration of human aging

In addition, the oxidative stress inhibitor 100 or 200 according to each of the embodiments and modifications thereof can play a role as an antioxidant agent or an agent for preventing (ameliorating) aging. Therefore, in the present specification, the oxidative stress inhibitor 100 or 200 can be replaced with an antioxidant agent 100 or 200, a hydroxyl radical inhibitor 100 or 200, or an agent for preventing (or ameliorating) aging 100 or 200.

### ANOTHER EMBODIMENT (4)

Therefore, other technical ideas derived from the first embodiment or each of the modifications of the first embodiment include at least the following (A) to (F) in addition to the technical ideas described above.
(A) An antioxidant agent containing silicon particles and silicon fine particles capable of generating hydrogen.
(B) The antioxidant agent set forth in (A), in which the ratio of the silicon fine particles having a crystallite diameter of less than 1 µm and aggregates of the silicon fine particles having a crystallite diameter of less than 1 µm to all the silicon particles, the silicon fine particles and the aggregates thereof is 5 mass% or less.
(C) The antioxidant agent set forth in (A) or (B), including a physiologically acceptable or medically acceptable metal element (iron (Fe)) supported on, or attached or adsorbed to at least a part of surfaces of the silicon particles or surfaces of silicon fine particles, or chemically bonded to the surfaces.
(D) A hydroxy radical inhibitor containing silicon particles and silicon fine particles capable of generating hydrogen.
(E) The hydroxy radical inhibitor set forth in (D), in which the ratio of the silicon fine particles having a crystallite diameter of less than 1 µm and aggregates of the silicon fine particles having a crystallite diameter of less than 1 µm to all the silicon particles, the silicon fine particles and the aggregates thereof is 5 mass% or less.
(F) The hydroxy radical inhibitor set forth in (D) or (E), including a physiologically acceptable or medically acceptable metal element (iron (Fe)) supported on, or attached or adsorbed to at least a part of surfaces of the silicon particles or surfaces of silicon fine particles, or chemically bonded to the surfaces.

Thus, according to the first embodiment or each of the modifications of the first embodiment, it is possible to provide an antioxidant agent or a hydroxyl radical inhibitor based on the above (A) to (F).

### ANOTHER EMBODIMENT (5)

Besides the technical ideas derived from the first embodiment or the modifications of the first embodiment described above and the technical ideas disclosed in (A) to (F) in the other embodiment (4) described above, the oxidative stress inhibitor, the antioxidant agent, and/or the hydroxyl radical inhibitor (hereinafter, referred to collectively as an "antioxidant agent" in the present embodiment) can also be applied in a method for producing an antibody drug. Therefore, in the present embodiment, an antioxidant agent containing silicon particles and silicon fine particles capable of generating hydrogen, based on the above-described technical ideas, can be used in the steps of a method for producing an antibody drug.

Specifically, in a method for producing an antibody drug according to the present embodiment, an oxidative stress inhibitor, an antioxidant agent and/or a hydroxy radical inhibitor containing the silicon particles and the silicon fine particles capable of generating hydrogen are used in at least one selected from the group of the following steps (I) to (IV):
(I) a step of preparing antibody-producing cells and/or plasma cells of a non-human animal, a human (only in the case of being ethically approved or permitted; the same applies hereinafter), a plant, bacteria (such as Escherichia coli), or any of eggs of various species;
(II) a step of separating the antibody-producing cells and/or the plasma cells;
(III) a hybridoma cell producing step of producing hybridoma cells; and
(IV) a monoclonal antibody producing step of producing a monoclonal antibody.

In the preparation step (I), the antioxidant agent according to the present embodiment is used, for example, when a non-human animal, a human, or a plant is immunized to trigger or induce an antibody response. The antioxidant agent can eliminate a part or substantially all of hydroxyl radicals that can be generated in the preparation step, so that it is possible to promote the efficiency of triggering or induction of the antibody response in the preparation step.

An example of the "non-human animal" is at least one animal selected from the group of a rat, a mouse, a monkey, sheep, a goat, a rabbit, a camel, a llama, an alpaca, a vicuna, a guanaco, a yak, a cattle, a muskox, a chiru, a raccoon dog, a mink, a sable, a common raccoon, a fox, a horse, a chinchilla, a dog, a pig and a cat. An example of the "plant" is at least one plant selected from the group of rice, corn and various oats (barley, wheat and rye).

In the separation step (II), the antioxidant agent according to the present embodiment can be used, for example, when antibody-producing cells (an example of B cells) and/or plasma cells (an example of B cells) are collected from an immunized non-human animal, a human, a plant, bacteria (such as Escherichia coli), or any of eggs of various species. Since the antibody-producing cells and/or the plasma cells can generate hydroxyl radicals, addition of oxidative stress to the cells may cause damage, death, decreased function and/or dysfunction of the cells. Thus, the antioxidant agent can eliminate a part or substantially all of hydroxyl radicals that can be generated in the separation step, so that it is possible to enhance the efficiency of collection of the antibody-producing cells and/or outer plasma cells in the separation step.

In the hybridoma cell producing step (III), the antioxidant agent according to the present embodiment can be used, for example, when the antibody-producing cells and/or the plasma cells described in the separation step (II) are fused with myeloma cells to produce hybridoma cells (fusion cells). Since the antibody-producing cells and/or the plasma cells can generate hydroxyl radicals, addition of oxidative stress to the cells may cause damage, death, decreased function and/or dysfunction of the cells, as in the separation step (II). Thus, the antioxidant agent can eliminate a part or substantially all of hydroxyl radicals that can be generated in the hybridoma cell producing step, so that it is possible to improve the efficiency of production of the antibody-producing cells and/or outer plasma cells in the hybridoma cell producing step.

In the monoclonal antibody producing step (IV), the antioxidant agent according to the present embodiment can be used, for example, when a monoclonal antibody is isolated from hybridoma cells that produce an antibody reactive specifically with an antigen (including a peptide and a recombinant protein). The antioxidant agent can eliminate a part or substantially all of hydroxyl radicals that can be generated in the monoclonal antibody producing step, so that it is possible to contribute to improvement of the function of the monoclonal antibody obtained in the monoclonal antibody producing step, and/or improvement of the yield of the monoclonal antibody.

The antioxidant agent according to the present embodiment can be applied in a monoclonal antibody purifying step of purifying a monoclonal antibody in addition to the monoclonal antibody producing step. Thus, it is possible to eliminate a part or substantially all of hydroxyl radicals that can be generated in the monoclonal antibody purifying step, so that the purification efficiency in the monoclonal antibody purifying step can be enhanced.

When a substantially neutral or weakly acidic solution preparation containing an antibody and a known isotonizing agent (sugar or salt), stabilizer, emulsifier and the like, the following method can be employed in a step using a liquid or a solution (typically an aqueous solution) (hereinafter, referred to collectively as a "solutions") among the above-described steps.

Specifically, the antioxidant agent (e.g. the antioxidant agent in a granular or lump form) can be brought into direct contact with the solution, where a medium, a covering material, a support, a carrier or the like is not interposed between the antioxidant agent and the solution.

In another example, a bag including a mesh filter permeable exclusively to the solution and containing the antioxidant agent so that the antioxidant agent does not diffuse into the solution can be disposed so as to contact the solution (e.g. the bag is disposed in a container containing the solution). For the antioxidant agent in this example, an aspect in which the particle size distribution of the antioxidant agent is adjusted by the classification step in the first embodiment is a preferred aspect.

In still another example, a nonwoven fabric (e.g. a part or the whole of the nonwoven fabric described in Japanese Design Registration No. 1561310) containing the antioxidant agent on a part of a surface and/or the inside thereof may be employed instead of the bag. In any of the cases described above, the bag or the nonwoven fabric is used in a clean state achieved by performing sterilization treatment or the like.

The antioxidant agent (e.g. the antioxidant agent in a granular or lump form) can also be used in a known bioreactor.

### ANOTHER EMBODIMENT (6)

Besides the technical ideas derived from the first embodiment or the modifications of the first embodiment described above and the technical ideas disclosed in (A) to (F) in the other embodiment (4) described above, the oxidative stress inhibitor, the antioxidant agent, and/or the hydroxyl radical inhibitor (hereinafter, referred to collectively as an "antioxidant agent" in the present embodiment) can also be applied in culture of cells in an in vitro fertilization and embryo transfer method or culture of at least one types of cells selected from the group of ES cells, iPS cells (induced pluripotent stem cells) and somatic stem cells.

Since the cells can generate hydroxyl radicals during culture of the above-described cells, addition of oxidative stress to the cells may cause damage, death, decreased function and/or dysfunction of the cells. Thus, the antioxidant agent can eliminate a part or substantially all of hydroxyl radicals that can be generated from the cells, so that it is possible to enhance the production efficiency or yield of the cells.

Specifically, as in the other embodiment (5), the following method can be employed in a step using a liquid or a solution (typically an aqueous solution) (hereinafter, referred to collectively as a "solutions") among the above-described steps in the process of culturing any of the above-described cells.

The antioxidant agent (e.g. the antioxidant agent in a granular or lump form) can be brought into direct contact with the solution (e.g. an in vitro development culture solution).

In another example, a bag including a mesh filter permeable exclusively to the solution and containing the antioxidant agent so that the antioxidant agent does not diffuse into the solution can be disposed so as to contact the solution (e.g. the bag is disposed in a container containing the solution). For the antioxidant agent in this example, an aspect in which the particle size distribution of the antioxidant agent is adjusted by the classification step in the first embodiment is a preferred aspect.

In still another example, a nonwoven fabric (e.g. a part or the whole of the nonwoven fabric described in Japanese Design Registration No. 1561310) containing the antioxidant agent on a part of a surface and/or the inside thereof may be employed instead of the bag. In any of the cases described above, the bag or the nonwoven fabric is used in a clean state achieved by performing sterilization treatment or the like.

As described above, the disclosure of each of the embodiments and modifications thereof is intended for explanation of those embodiments or modifications, and is not intended to limit the present invention. In addition, claims also include other modifications within the scope of the present invention including other combinations of the embodiments and modifications thereof.

### INDUSTRIAL APPLICABILITY

The oxidative stress inhibitor of the present invention can be widely used in various industries including pharmaceutical industry (including quasi-pharmaceutical products) and medical industry in which hydrogen is applied, and food industry.

### DESCRIPTION OF REFERENCE SIGNS

10: Silicone particles, silicon fine particles
20: Metal element
100, 200: Oxidative stress inhibitor, antioxidant agent, hydroxyl radical inhibitor, agent for preventing (ameliorating) aging

## Claims

1. An oxidative stress inhibitor comprising silicon particles and silicon fine particles capable of generating hydrogen.

2. The oxidative stress inhibitor according to claim 1, wherein a ratio of the silicon fine particles having a crystallite diameter of less than 1 µm and aggregates of the silicon fine particles having a crystallite diameter of less than 1 µm to all the silicon particles, the silicon fine particles and the aggregates thereof is 5 mass% or less.

3. The oxidative stress inhibitor according to claim 1 or 2, comprising iron (Fe) supported on, or attached or adsorbed to at least a part of surfaces of the silicon particles or silicon fine particles, or chemically bonded to the surfaces.

4. The oxidative stress inhibitor according to claim 3, wherein a mass ratio of the iron (Fe) is 0.1 ppmw or more and 1000 ppmw or less when the silicon particles or the silicon fine particles is assumed to be 1.

5. The oxidative stress inhibitor according to claim 3, wherein a mass ratio of the iron (Fe) is 0.5 ppmw or more and 1000 ppmw or less when the silicon particles or the silicon fine particles is assumed to be 1.

6. A supplement comprising the oxidative stress inhibitor according to any one of claims 1 to 5.

7. A supplement for preventing or ameliorating a skin disease, the supplement comprising the oxidative stress inhibitor according to any one of claims 1 to 5.

8. The supplement for preventing or ameliorating a skin disease according to claim 7, wherein the skin disease is at least one selected from the group of eczema, inflammatory skin disease, allergic dermatitis with impaired skin barrier function, and atopic dermatitis.

9. A supplement for preventing or ameliorating constipation, diarrhea or hangover syndrome, the supplement comprising the oxidative stress inhibitor according to any one of claims 1 to 5.

10. A supplement for preventing or ameliorating aging, the supplement comprising the oxidative stress inhibitor according to any one of claims 1 to 5.

11. A food product comprising the oxidative stress inhibitor according to any one of claims 1 to 5.

12. An antioxidant agent comprising silicon particles and silicon fine particles capable of generating hydrogen.

13. The antioxidant agent according to claim 12, wherein a ratio of the silicon fine particles having a crystallite diameter of less than 1 µm and aggregates of the silicon fine particles having a crystallite diameter of less than 1 µm to all the silicon particles, the silicon fine particles and the aggregates thereof is 5 mass% or less.

14. The antioxidant agent according to claim 12 or 13, comprising iron (Fe) supported on, or attached or adsorbed to at least a part of surfaces of the silicon particles or silicon fine particles, or chemically bonded to the surfaces.
